# EUROPEAN PATENT APPLICATION

(11) **EP 4 292 516 A1**
(43) Date of publication of application: **20.12.2023**
(21) Application number: 22179491.0
(22) Date of filing: 16.06.2022
(51) Int. Cl.: A61B 5/00, A61B 5/01

(54) **PHOTOACOUSTIC DETECTING DEVICE FOR MEASURING A PARAMETER OF INTEREST IN A MEDIUM**

(71) Applicant: Eclypia, 38000 GRENOBLE (FR); Commissariat à l'énergie atomique et aux énergies alternatives (CEA), 75015 Paris (FR)
(72) Inventor: GOUJON, Charles-élie, 38054 Grenoble cedex 09 (FR); LARTIGUE, Olivier, 38054 Grenoble cedex 09 (FR); GREFFIER, Damien, Patrice, 49440 Challain-La-Potherie (FR); PALLIER, Cédric, Gwanael, 35470 BAIN DE BRETAGNE (FR); COUTARD, Jean-Guillaume, 38660 Saint-Pancrasse (FR); BERNET, Florent, 38100 Grenoble (FR)
(74) Representative: Fidal Innovation

(57) **Abstract**

A photoacoustic detecting device (1) for measuring a parameter of interest in a medium (M) comprising:
➢ a housing (7) formed by a baseplate (72) and a cover,
➢ a light source (2), configured to emit a light signal,
➢ a photoacoustic cell (9) comprising a hollow contact surface (91) intended to be in contact with the medium (M), where the light signal is able to propagate in the photoacoustic cell (9) and pass through the hollow contact surface (91) to reach the medium (M),
➢ a guiding element (8), configured to direct the light signal toward the photoacoustic cell (9),
➢ a transducer (3) assembled to the photoacoustic cell (9) and configured to detect a generated signal, where the generated signal is generated in the photoacoustic cell (9) by a photothermic effect in the medium (M) in response to an irradiation of the medium (M) by the light signal,
➢ A maintaining element configured to assemble the light source (2), the transducer (3), the photoacoustic cell (9) and said guiding element (8) in a single building block,
➢ said single building block is provided in the housing (7) such that the hollow contact surface (91) of the photoacoustic cell (9) emerges out of an aperture (721) of the baseplate (72),
wherein
➢ the single building block is freely received in the housing (7), and in that
➢ the detecting device (1) comprises elastic components (110, 120), configured to elastically deform under the effect of a movement of the single building block relatively to the housing (7)

## Description

### FIELD OF THE INVENTION

The present invention relates to photoacoustic detecting devices for measuring a parameter of interest in a medium.

### BACKGROUND OF THE INVENTION

Photoacoustic detection may be used in the field of detecting devices, notably for detecting parameters of interest such as chemical components in a medium. The medium may be an organic tissue, such as the skin of a human.

The photoacoustic detection is based on the irradiation of a medium M to be analysed by a light signal emitted by a light source. Generally, the light source is one or several lasers, in particular a quantum cascade laser (QCL). The light signal is a light beam of a chosen wavelength. The wavelength is chosen regarding the type of parameter of interest to be measured.

The photoacoustic detection is based on the detection of a pressure wave associated with a thermic wave. The thermic wave is generated under the effect of the absorption of the light beam by the medium. Such absorption creates a local heating of chemical components in the medium, where the light beam has been absorbed. The thermic wave propagates in the medium M before propagating outside the medium. More precisely, when the thermic wave comes out of the medium, after its propagation, a pressure wave is generated, which can be detected.

The photoacoustic detection may be made specific to particular chemical components, by adjusting the wavelength and/or the modulation frequency of the light beam. More precisely, the wavelength may be adjusted to match an absorption peak of the component to analyse.

Photoacoustic detection then is a non-invasive way of analysing a medium of interest. Numerous photoacoustic detecting devices have been developed.

For example, as described in document EP3885765, such a detecting device is intended to be worn by a person, for example on the arm of a person. The medium to be analysed is typically the skin of the person. The device comprises a contact face, intended to be applied, in contact, with the skin of the person wearing the device. The device generally comprises a hollow cavity emerging onto an aperture in the contact face. A light signal is emitted and passes through the cavity to reach the skin. An acoustic transducer is provided to detect a photoacoustic wave extending through the cavity, which is then analysed to detect a parameter of interest, such as glucose concentration in the medium.

One challenge with such devices is that the contact face must stay in contact with the medium to be analysed to avoid any measure artefact. However, when such a device is worn by a person, the contact may be lost at some point, due to some movements of the person.

There is then a need to provide a photoacoustic detecting device with a contact face that would stay in permanent contact with the medium to be analysed.

### BRIEF SUMMARY OF THE INVENTION

Thus, the invention relates to a photoacoustic detecting device for measuring a parameter of interest in a medium comprising:
➢ a housing formed by a baseplate and a cover,
➢ a light source, configured to emit a light signal,
➢ a photoacoustic cell comprising a hollow contact surface intended to be in contact with the medium, where the light signal is able to propagate in the photoacoustic cell and pass through the hollow contact surface to reach the medium,
➢ a guiding element, configured to direct the light signal toward the photoacoustic cell,
➢ a transducer assembled to the photoacoustic cell and configured to detect a generated signal, where the generated signal is generated in the photoacoustic cell by a photothermic effect in the medium in response to an irradiation of the medium by the light signal,
➢ one or more maintaining element configured to assemble the light source, the transducer, the photoacoustic cell and said guiding element in a single building block,
➢ said single building block is provided in the housing such that the hollow contact surface of the photoacoustic cell emerges out of an aperture of the baseplate, characterized in that
➢ the single building block is freely received in the housing, and in that
➢ the detecting device comprises elastic components, configured to elastically deform under the effect of a movement of the single building block relatively to the housing.

Thanks to these provisions, the configuration of the detecting device allows to efficiently compensate movement of the single building block relatively to the housing, therefore allowing accurate measurement of the parameter of interest by avoiding any artefact measure. Indeed, since the photoacoustic cell emerges out of an aperture of the baseplate, a movement of the medium against the photoacoustic cell may cause the photoacoustic cell to displace, for example such movement may push the photoacoustic cell inside the housing where the detection can no longer be made with accuracy. The elastic components allow to compensate for such displacement of the photoacoustic cell, such that the photoacoustic cell is able to stay in contact with the medium at any time.

In an embodiment, the photoacoustic cell emerges out of the baseplate for about 0.5 to 1mm.

According to different aspects, it is possible to provide the one and / or the other of the characteristics below taken alone or in combination:

Said elastic components comprise at least one elastic element being in contact with an interior surface of the cover and the single building block.

The elastic element allows to compensate a movement between the cover and the single building block. Typically, such movement may be induced by an upward force pushing the single building block towards the cover.

The at least one elastic element is a leaf spring.

The fabrication is facilitated since leaf springs are used as components in the electronic field.
the at least one elastic element is a spiral spring received in a spring guide.
the at least one elastic element comprises a layer of an elastic material.

Numerous different types of elastic elements may be used.
the at least one elastic element is attached to the maintaining element, said maintaining element being a printed card board (PCB), said printed card board facing the interior surface of the cover.

The attachment of the elastic elements directly to the PCB allows an easy fabrication since the machinery used to construct the PCB can be used to install the elastic elements on the PCB. The installation can then be made in one single step. Furthermore, no fixing elements such as glue or screws need to be used to fix the at least one elastic element to the PCB. This is especially the case when the elastic elements are leaf springs.
the elastic components comprise a layer of an elastic material provided between an interior surface of the baseplate and the single building block.

The provision of the layer of elastic material allows to compensate a movement between the baseplate and the building block. Typically, such movement may be induced by an upward force pushing the building block towards the cover. Such movement may also be induced by a downward force pushing the building block towards the baseplate, where the downward force is, for example, applied by the elastic elements.
the layer of elastic material is configured to elastically deform according to an elongation and a compression.

As stated above, the layer of elastic material is typically configured to compensate a movement of the building block induced by an upward and/or a downward force applied on the building block. The thickness of the layer of elastic material is chosen to compensate for a movement of the building block of an amplitude of a few millimetres.

the layer of elastic material is placed between the interior surface of the baseplate and the light source, said elastic material being configured to conduct heat emitted by the medium to the light source.

A thermal conductivity is then created between the medium and the light source, which is particularly advantageous when the light source needs to function under specific functioning temperatures. The layer of elastic material may then comprise two different technical functions.

In an embodiment, the housing comprises two fins, symmetrically disposed with regards to the aperture of the baseplate, wherein said fins are configured to come in contact with the medium to be analysed, such that the two fins are configured to reduce any convection effect that could be induced by the passage of the external air between the baseplate and the medium to be analysed.
the elastic material is a layer of gel.
the layer of elastic material is a putty of thermal silicone.
in an embodiment, the elastic material is a copper coolant cable.

The above characteristics typically allow an easy and relatively costless fabrication of the device. No further fixing elements may be needed between the baseplate and the layer of elastic material.

Moreover, it allows thermal continuity both in extension and in compression.
the elastic material is received in a recess of the interior surface of the baseplate.

This is advantageous regarding the compactness of the device.
the parameter of interest comprises a chemical component such as glucose, cholesterol, triglyceride, urea, albumin and/or alcohol.

The detecting device is efficient to detect numerous parameters of interest.

### BRIEF DESCRIPTION OF THE DRAWINGS

Embodiments of the invention will be described below with reference to the drawings, described briefly below:
[Fig. 1] represents a photoacoustic detecting device worn on the arm of a person P.
[Fig. 2] is a block schema of a photoacoustic detecting device.
[Fig. 3] is a schematic representation of a photoacoustic detecting device according to an embodiment.

In the drawings, identical references designate identical or similar objects.

### DETAILED DESCRIPTION OF THE INVENTION

The invention relates to a photoacoustic detecting device 1 for measuring a parameter of interest in a medium M.

In a non-limiting example, the photoacoustic detecting device 1 (or "detecting device 1" in the following description) is intended to be worn by a person P. The medium M may be an organic tissue such as the skin of the person P wearing the detecting device 1.

A parameter of interest may be a chemical component present in the skin of the person P, such as molecules. The parameter of interest may comprise glucose, cholesterol, triglyceride, urea, albumin, and/or alcohol. This list is non exhaustive and several other parameters of interest may be measured.

The measured parameters may then be analysed to determine a blood concentration of glucose, cholesterol and so on.

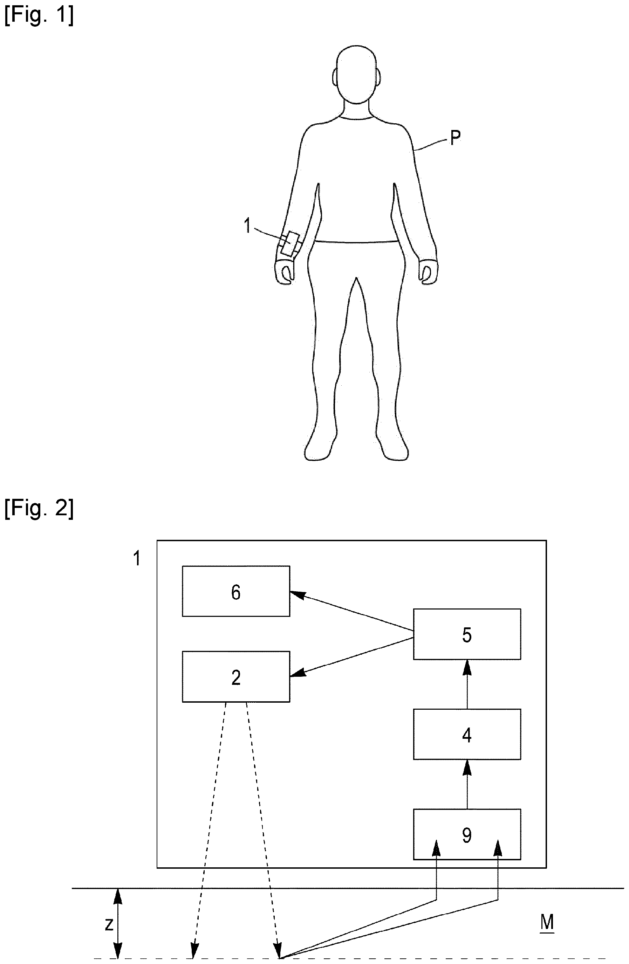

As seen on figure 1, the detecting device 1 may be worn on the arm, or wrist, of a person P. The detecting device 1 may be fixed to the arm of the person P by means of a bracelet.

The detecting device 1 may allow a continuous monitoring of the person P, by repeatedly measuring the parameters of interest of the person P, while it is worn.

Figure 2 is a block diagram of the detecting device 1.

The detecting device 1 comprises :
- at least one light source 2, configured to emit a light signal.
- a transducer 3 acquiring a signal coming from the medium M,
- a signal processing module 4 for analysing the signal detected by the transducer.

In an embodiment, the transducer 3 may be an acoustic transducer 3 detecting an acoustic signal generated in response to the irradiation of the medium M by the light signal

The transducer 3 can be connected to the signal processing module 4 such that the signal processing module 4 receives a signal coming from the transducer 3.

In a non-limiting embodiment, the signal processing module 4 may comprise an analog-to-numeric converter, converting the signal acquired by the transducer 3 to a numeric signal

In another embodiment, the transducer 3 may directly transmit a numeric signal to the signal processing module 4.

The signal processing module 4 may be implemented in a processor (not shown) which may or not be remote from the detecting device 1.

The detecting device 1 may comprise other components. For example, the detecting device 1 may also comprise an adapting module 5 for adapting irradiation parameters of the light source 2 and a memory 6, for example. These components won't be further described.

In an embodiment, the light source 2 emits a light signal at a chosen wavelength, towards the medium M to be analysed. The chosen wavelength may be chosen according to the parameters of interest to be measured.

More precisely, the wavelength may correspond to the absorption peak of the parameter of interest to be measured. As an example, to detect glucose, the wavelength may be 1034 cm⁻¹, which corresponds to the absorption peak of the glucose.

The light source may be a laser, and more particularly a quantum cascade laser (QCL). In a variant embodiment, the light source 2 may be an electroluminescent diode (led).

In an embodiment, the detecting device 1 may comprise a plurality of light sources 2. In this embodiment, each light source 2 may emit a light signal at different wavelength. In variant, some light sources 2 emit a light signal at the same wavelength, while other light sources 2 emit a light signal at a different wavelength.

In the following description, the detecting device 1 is described as comprising only one light source 2, while it is understood that the same specification applies for a plurality of light sources 2.

The light signal emitted by the light source 2 propagates to the medium M and through it. This phenomenon is represented by the dotted arrows on figure 2. The light signal is absorbed by the constituents of the medium M under a depth z depending on the chosen wavelength of the light signal, the frequency of the light signal and of the composition of the medium M.

The absorption of the light signal energy causes a local heating of the medium M. As a consequence, a thermic signal propagates in the medium M (phenomenon illustrated by the full arrows on figure 2), in particular towards the surface of the medium M. Moreover, the thermic wave may create, outside the medium M, a pressure wave that propagates outside the medium M. Such a pressure wave may be detected by the acoustic transducer 3.

In an embodiment, the thermic wave may also be detected by means of a thermic transducer, such as a thermometer, not shown in the figures.

The detecting device 1 may comprise an acoustic and/or a thermic transducer 3. Other types of transducer 3 may be used.

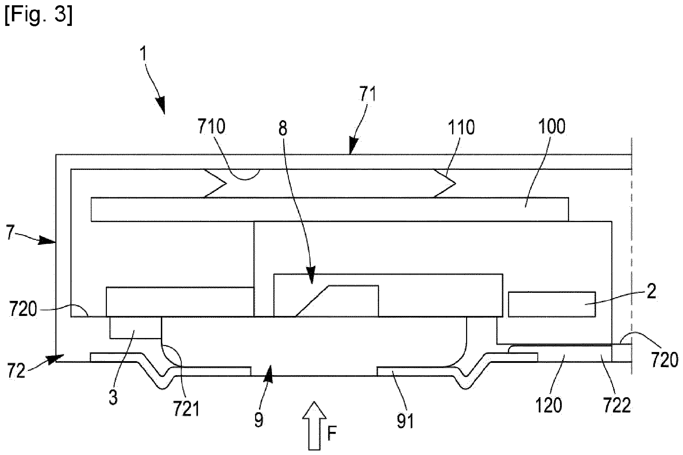

Figure 3 schematically illustrates a detecting device 1 according to an embodiment.

The detecting device 1 may comprise a housing 7, formed by a cover 71 and a baseplate 72. The housing 7 comprises an interior delimited by an interior surface 710 of the cover 71 and an interior surface 720 of the baseplate 72.

The interior of the housing 7 houses a light source 2, which, as described above, may be a QCL.

A guiding element 8 is also provided. The guiding element 8 is configured to direct the light signal emitted by the light source 2 towards the medium M to be analysed.

In the embodiment in which the detecting device 1 comprises a plurality of light sources 2, the guiding element 8 may also be configured to collimate each light signal emitted by each light source 2 into a single light signal directed towards the medium M to be analysed.

More particularly, the guiding element 8 is configured to direct the light signal emitted by the light source 2 through a photoacoustic cell 9 (or "cell 9" in the following description), placed between the medium M to be analysed and the light source 2.

The cell 9 extends from the guiding element 8 to a hollow contact surface 91 of said cell 9 through which the medium M is accessible. The hollow surface contact 91 may generally present the shape of a ring. The hollow contact surface 91 may have the shape of a circle and the hollow may be positioned on the centre of the circle and may present also a general circular shape. Other configurations are possible.

The hollow part of the hollow contact surface 91 may be closed by a window of transparent material, allowing the light signal to traverse it.

The hollow surface contact 91 may be intended to be placed against the medium M to be analysed, and more precisely may be placed in direct contact with the medium M to be analysed.

Then, when the detecting device 1 is worn by a person P, as represented on figure 1, the hollow contact surface 91 is directly placed against the skin of the person P.

To this end, the base plate 72 comprises an aperture 721 from which the hollow contact surface 91 emerges. Thus, the hollow contact surface 91 of the cell 9 is positioned outside the interior of the housing 7 and can be placed in direct contact with the medium M to analyse.

Regarding the photoacoustic detection, a thermic wave is generated under the effect of the absorption by the medium of the light beam emitted by the light source. Such absorption creates a local heating of chemical components in the medium, where the light beam has been absorbed. The thermic wave propagates in the medium M before propagating outside the medium. When the thermic wave comes out of the medium, after its propagation, a pressure wave is generated, which propagates in the cell 9 where it can be detected. The pressure wave, i.e. an acoustic wave, is then induced by the photothermic effect.

The transducer 3 is located in the interior of the housing 7 and is preferably assembled to the cell 9, such that the transducer 3 can detect and measure the generated pressure wave.

As stated above, to allow an accurate detection of the parameter of interest, and to avoid any measure artefacts, the hollow contact surface 91 of the cell 9 must remain in contact with the medium M to analyse at each time.

This can be particularly difficult when the detecting device 1 is intended to be worn on the arm of a person P, as illustrated on figure 1. Indeed, an arm is not static and any movement of the user could cause a displacement of the detecting device 1, leading to a loss of contact between the hollow contact surface 91 and the skin of the person P. Moreover, the texture itself of the skin could cause some loss of contact, since the skin is, per nature, soft and elastic.

It is also important since the underlying skin mapping is an important parameter for the accuracy of the measurement. If the device moves, the underlying skin may have a different architecture, and therefore new measurements may not be comparable with old measurements.

To solve this technical problem, the detecting device 1 is provided with one or several maintaining elements. Such maintaining element 100 may be a metallic plate, a plastic plate etc. The maintaining element is used to maintain at least the light source 2, the transducer 3, the guiding element 8 and the cell 9 together. More precisely, the light source 2, the transducer 3, the guiding element 8 and the cell 9 together are attached together via one or more maintaining element, to form one single building block. The single building block is considered cinematically as a rigid body in normal use.

In a preferred embodiment, the maintaining element 100 is one or more printed cardboard(s) (PCB) 100. In the following description, it is considered that the maintaining element is a PCB 100, while other applications and embodiments enter within the scope of the present application.

The PCB also ensures the electronic and/or electric connections between the components of the building block.

The building block is freely received in the housing 7. By "freely received", it means that the building block is neither attached to the baseplate 72, nor to the cover 71. In other words, there is no fixing element, such as screw, glue etc., that fixes the building block to the housing 7.

In the embodiment represented on figure 3, the components bloc comprises, from bottom to top, where the bottom is situated near the baseplate 72 and the top near the cover 71, the cell 9, where the hollow surface contact 91 of the cell 9 emerges out of the baseplate 72, the transducer 3, the guiding element 8, the light source 2 and a PCB 100 provided at the top.

Other PCBs or other types of maintaining elements may be provided.

The PCB 100 faces the interior surface 710 of the cover 71. Elastic elements 110 are provided between the PCB 100 and the interior surface 710 of the cover 71. Such elastic elements 110 are able to elastically deform themselves under the effect of a movement of the building block relatively to the housing 7.

More precisely, in the "at rest" state, where the building block is received in the housing 7 without any forces applied on it, for example in the state where the detecting device 1 is not worn by a person P, the elastic elements 110 may be in their rest state in which they are neither elongated nor contracted.

The elastic elements 110 may comprise leaf springs, spiral springs potentially received in a guiding element to ensure a translation movement according to an only up-down direction, pistons and/or gel.

The detecting device 1 may comprise at least one elastic element 110. Advantageously, the detecting device 1 comprises more than one elastic element 110.

The one or more elastic element(s) 110 may be disposed to compensate for a movement of the single building block. As a non-limitative example, the one or more elastic elements 110 may be disposed symmetrically regarding an axis of measure passing by the centre of and being normal to the hollow contact surface 91.

In an embodiment, the elastic elements 110 are fixed to the PCB 100. In another embodiment, the elastic elements 110 are fixed to the interior surface 710 of the cover 71.

The detecting device 1 may also comprise a layer of elastic material 120 between the building block and the interior surface 720 of the base plate 71. Such a layer of elastic material 120 also is able to elastically deform under the effect of a movement of the building block relative to the housing 7.

Such a layer of elastic material 120 may be a layer gel. Advantageously, the layer of elastic material 120 is a putty of silicone.

A movement of the building block could be induced by an upward force F applied to the surface contact 91 of the cell 9. Such upward force F is represented by the arrow on figure 3. Such upward force would cause an upward displacement of the whole building block relatively to the housing 7.

The elastic elements 110 would absorb the energy generated by the displacement of the components bloc toward the top of the housing by deforming elastically. The movement of the building block therefore can be compensated, allowing the hollow contact surface 91 of the cell 9 to stay in contact with the medium M.

When the upward force F is applied, the building block will move upwards, towards the interior surface 710 of the cover 71, thereby deforming the elastic elements 110 by compressing them between the PCB 100 and the interior surface 710 of the cover 71. The elastic elements 110 however will not deform completely beyond their elastic limit but will resist, pushing back the building block towards the baseplate 72. Moreover, the layer of elastic material 120 may be able to also deform elastically, by elongating itself according to the upward movement of the single building block. The elastic elements 110 and the layer of elastic material 120 then allow the compensation of the movement of the building block relatively to the housing 7.

Once the force F stops to be applied, the elastic elements 110 return to their rest state, as well as the building block which lays on the interior surface 720 of the baseplate 72.

When the building block returns to its rest state, the layer of elastic material 120 may compensate for the downward movement of the building block by deforming itself elastically in a contracted state.

When the building block is in its rest state, there is no more elastic deformation of the elastic elements 110 or the layer of elastic material 120.

Another challenge of a photoacoustic detecting device using a QCL as a light source 2 is that the QCL has an optimal functioning temperature, typically comprised between 30°C and 40°C for this application. When the detecting device 1 is applied against the skin of a person P, the corporal temperature of the person P may be used as a heating source for the QCL. A thermic exchange may then take place between the skin and the QCL.

The elastic material used in the layer of elastic material 120 may then present good thermal conductivity properties. Consequently, the layer of elastic material 120 may be a layer of thermic gel, such as a thermic putty of silicone.

The layer of elastic material 120 may typically be placed between the interior surface 720 of the baseplate 72 and the building block, where the light source 2 is situated, as visible on figure 3.

The layer of elastic material 120 may then present both functions of compensating movement of the single building block and allowing the light source 2 to work properly. The layer of elastic material 120 may then enhance the general functioning of the detecting device 1.

Advantageously, the elastic elements 110 and the layer of elastic material 120 may allow the building block to have a movement of an amplitude between 1 and 2 mm. The person of the art is skilled to choose elastic elements presenting an elasticity and a thickness of the layer of elastic material allowing to reach this result.

Furthermore, to render the detecting device 1 more compact, the layer of elastic material 120 may be provided in a recess 722 of the interior surface 720 of the baseplate 72. Preferably, the recess 722 presents the same thickness as the thickness of the layer of elastic material 120.

While exemplary embodiments of the invention has been described, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiment disclosed as the best mode contemplated for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

## Claims

1. Photoacoustic detecting device (1) for measuring a parameter of interest in a medium (M) comprising:
➢ a housing (7) formed by a baseplate (72) and a cover,
➢ a light source (2), configured to emit a light signal,
➢ a photoacoustic cell (9) comprising a hollow contact surface (91) intended to be in contact with the medium (M), where the light signal is able to propagate in the photoacoustic cell (9) and pass through the hollow contact surface (91) to reach the medium (M),
➢ a guiding structure (8), configured to direct the light signal toward the photoacoustic cell (9),
➢ a transducer (3) assembled to the photoacoustic cell (9) and configured to detect a generated signal, where the generated signal is generated in the photoacoustic cell (9) by a photothermic effect in the medium (M) in response to an irradiation of the medium (M) by the light signal,
➢ one or more maintaining element(s) (100) configured to assemble the light source (2), the transducer (3), the photoacoustic cell (9) and said guiding element (8) in a single building block,
➢ said single building block is provided in the housing (7) such that the hollow contact surface (91) of the photoacoustic cell (9) emerges out of an aperture (721) of the baseplate (72),
**characterized in that**
➢ the single building block is freely received in the housing (7), and **in that**
➢ the detecting device (1) comprises elastic components (110, 120), configured to elastically deform under the effect of a movement of the single building block relatively to the housing (7).

2. Device (1) according to claim 1, wherein said elastic components comprise at least one elastic element (110) being in contact with an interior surface of the cover and the single building block.

3. Device (1) according to claim 2, wherein the at least one elastic element (110) is attached to the maintaining element, wherein the maintaining element is a printed cardboard.

4. Device (1) according to claim 2 or 3, wherein the at least one elastic element (110) comprises a leaf spring.

5. Device (1) according to any of claims 2 to 4, wherein the at least one elastic element (110) comprises a spiral spring received in a spring guide.

6. Device (1) according to any of claims 2 to 5, wherein the at least one elastic element (110) comprises a layer of an elastic material.

7. Device (1) according to any of claims 1 to 6, wherein the elastic components comprise a layer of an elastic material provided between an interior surface of the baseplate (72) and the single building block.

8. Device (1) according to claim 7, wherein the layer of elastic material (120) is configured to elastically deform according to an elongation and a compression.

9. Device (1) according to claims 7 or 8, wherein the layer of elastic material (120) is placed between the interior surface of the baseplate (72) and the light source (2), said elastic material being configured to conduct heat emitted by the medium (M) to the light source (2).

10. Device (1) according to any of claims 7 to 9, wherein the elastic material is a layer of gel.

11. Device (1) according to any of claims 7 to 10, wherein the layer of elastic material (120) is a putty of thermal silicone.

12. Device (1) according to any of claims 7 to 11, wherein the layer of elastic material (120) is received in a recess of the interior surface of the baseplate (72).
